# EUROPEAN PATENT APPLICATION

(11) **EP 4 296 319 A1**
(43) Date of publication of application: **27.12.2023**
(21) Application number: 21926761.4
(22) Date of filing: 09.12.2021
(51) Int. Cl.: C09C 1/40, C09C 1/64, C09C 3/04, C09C 3/06, C09C 3/08, A61Q 1/02, A61K 8/25, A61K 8/26, G03G 9/09

(54) **PIGMENT, COSMETIC PREPARATION, INK, COATING MATERIAL, TONER AND MOLDED ARTICLE**

(30) Priority: 18.02.2021 JP 2021024125
(71) Applicant: DIC CORPORATION, Itabashi-ku Tokyo 174-8520 (JP)
(72) Inventor: MIYOSHI Ayaka, Kamisu-shi, Ibaraki 314-0193 (JP); KAICHI Hideo, Kamisu-shi, Ibaraki 314-0193 (JP); WATANABE Takanori, Kamisu-shi, Ibaraki 314-0193 (JP); KUDOU Arata, Kamisu-shi, Ibaraki 314-0193 (JP)
(74) Representative: TBK
(86) International application number: PCT/JP2021/045263
(87) International publication number: WO 2022/176336

(57) **Abstract**

An object to be achieved by the present invention is to provide a pigment which is comfortable and smooth to the touch in a coating, spreads well on the skin, for example, when used in a cosmetic material such as a foundation or an eye shadow, and exhibits good color development and gloss. A pigment of the present invention is a coated pigment formed by coating a surface of a flaky base material made of at least one metal or metal oxide with a colorant. The surface of the flaky base material has a coarse particle area fraction of 9% or less. Further, it is preferable that the flaky base material be at least one or more selected from the group consisting of mica, aluminum, alumina, and glass.

## Description

### Technical Field

The present invention relates to a pigment and a cosmetic material, an ink, a coating material, a toner, or a molded article that contains the pigment.

### Background Art

In the related art, in the field of cosmetic materials, inks, and coating materials, cosmetic materials, inks, and coating materials are required to exhibit good color development and gloss when applied and to have excellent brightness such as a glittering appearance. Particularly, makeup cosmetic materials such as lipsticks, eye shadows, blushers, and nail cosmetics are required to exhibit uniform color development in order to provide a better finish when applied to the skin. At the same time, makeup cosmetic materials may also be required to have a glittering appearance for reasons such as strongly reflecting light and thereby showing a more beautiful finish like pearls and metal surfaces. Under these circumstances, in cosmetic materials, inks, coating materials, toners, or molded articles, organic pigments and sparkling materials such as mica are used alone or in combination as pigment components.

For example, PTL 1 describes a flaky pigment having an average particle diameter of 5 to 60 um in which composite particles based on an ordered mixture are formed by performing a high-speed stirring treatment on a mixture of a flaky substrate material made of flaky substrate particles having an aspect ratio of 10 to 120 and a pigment and/or a dye made of particles having an average particle diameter of 5 um or less without using a liquid medium.

Further, PTL 2 describes a cosmetic material prepared by blending, into a cosmetic base, at least one of a colored inorganic powder or a colored pearlescent pigment obtained by strongly fixing Red No. 226 to a surface of an inorganic powder or a pearlescent pigment.

### Citation List

### Patent Literature

PTL 1: Japanese Unexamined Patent Application Publication No. 05-214257
PTL 2: Japanese Unexamined Patent Application Publication No. 62-169716

### Summary of Invention

### Technical Problem

For the pigments described in PTLs 1 and 2, the flaky substrate material is simply coated with a colorant, and the condition of the pigment surface is not taken into consideration at all. Such pigments in the related art have a problem in that color unevenness may occur because these pigments have large surface unevenness due to uneven coating of the flaky base material with the colorant and thus exhibit decreased smoothness, for example, when used in a cosmetic material and applied to the skin. Therefore, there is a demand for a coated pigment that is comfortable to the touch, spreads well, and is unlikely to cause color unevenness.

An object of the present invention is to solve the above-described problem with coated pigments and to provide a pigment which is comfortable and smooth to the touch in a coating, spreads well on the skin, for example, when used in a cosmetic material such as a foundation or an eye shadow, and exhibits good color development and gloss.

### Solution to Problem

As a result of intensive research, the present inventors found that the above-described problem can be solved by controlling the coarse particle area fraction of the surface of the flaky base material, thereby completing the present invention.

That is, the present invention relates to the following items:
"Item 1. A pigment which is a coated pigment formed by coating a surface of a flaky base material made of at least one metal or metal oxide with a colorant, wherein the surface of the flaky base material has a coarse particle area fraction of 9% or less.
Item 2. The pigment according to Item 1, wherein the flaky base material is at least one or more selected from the group consisting of mica, aluminum, alumina, and glass.
Item 3. The pigment according to Item 1 or 2, including at least one or more organic pigments as the colorant.
Item 4. The pigment according to any one of Items 1 to 3, wherein the surface of the flaky base material is further coated with a layer containing, as a component, at least one or more selected from the group consisting of a resin, a metal, and a metal oxide.
Item 5. The pigment according to any one of Items 1 to 4, wherein the pigment is further coated with a layer containing, as a component, at least one or more selected from the group consisting of a resin, a metal, and a metal oxide.
Item 6. A cosmetic material, an ink, a coating material, a toner, or a molded article, including the pigment according to any one of Items 1 to 5."

### Advantageous Effects of Invention

The pigment of the present invention is comfortable and smooth to the touch in a coating, spreads well when applied, and also exhibits good color development and gloss. Therefore, the pigment spreads well on the skin, particularly when used in a cosmetic material such as a foundation or an eye shadow, exhibits good color development and gloss, and has excellent brightness such as a glittering appearance.

### Brief Description of Drawings

[Fig. 1] Fig. 1 is a SEM micrograph showing a coated pigment (1) in the Examples.
[Fig. 2] Fig. 2 is a SEM micrograph of a coated pigment (7) in the Examples.
[Fig. 3] Fig. 3 is a black and white binarized image of Fig. 1.
[Fig. 4] Fig. 4 is a SEM image showing an example of a pigment of the present invention (the frame in the micrograph shows an example of a coarse particle area fraction calculation area).

### Description of Embodiments

Hereinafter, the present invention will be described in detail.

### [Pigment]

A pigment of the present invention is a pigment formed by coating a surface of a flaky base material made of at least one metal or metal oxide with a colorant, and the surface of the base material has a coarse particle area fraction of 9% or less. Hereinafter, the pigment of the present invention will be referred to as "coated pigment". In the present invention, "coated" includes not only a case where the surface of the flaky base material is completely coated but also a case where only part of the surface is coated (for example, "deposition").

The coarse particle area fraction of the coated pigment in the surface is 9% or less, preferably 6% or less, more preferably 5% or less, still more preferably 4% or less, and most preferably 3% or less. In a case where the coarse particle area fraction thereof is in the above-described ranges, friction on the coated pigment or on the surface of a coating containing the coated pigment can be reduced, and the pigment is comfortable and smooth to the touch. In the present invention, "coarse particle" denotes a particle that is sufficiently smaller than the flaky base material, for example, a particle having a major diameter of about 0.01 um to about 3 um as observed with a scanning electron microscope, or a particle having a particle diameter of about 0.05% to about 15% of the particle diameter of the flaky base material. The coarse particle area fraction can be determined by observing the surface of one coated pigment with a scanning electron microscope and calculating the percentage of the area of the coarse particles present on the surface thereof.

### (Flaky base material)

The flaky base material of the coated pigment is made of at least one kind of metal or metal oxide. Examples of the metal include silicon (Si), iron (Fe), aluminum (Al), sodium (Na), calcium (Ca), magnesium (Mg), potassium (K), copper (Cu), manganese (Mn), titanium (Ti), silver (Ag), gold (Au), platinum (Pt), lead (Pb), chromium (Cr), tin (Sn), molybdenum (Mo), gallium (Ga), and indium (In). Further, examples of the metal oxide include oxides of these metals.

Examples of the flaky base material include mica, aluminum, alumina, glass, titanium dioxide, red iron oxide, iron oxide, yellow iron oxide, black iron oxide, zinc oxide, Prussian blue, ultramarine blue, chromium oxide, kaolin, clay, bentonite, bismuth oxychloride, zirconium oxide, and magnesium oxide. Among the flaky base materials, at least one or more selected from the group consisting of mica, aluminum, alumina, and glass are preferable.

Examples of the mica include natural mica, synthetic mica, synthetic phlogopite, titanated mica, synthetic titanated mica, iron oxide-coated mica, iron oxide-coated synthetic mica, and chromium hydroxide mica. Examples of the aluminum (Al) include, in addition to elemental aluminum, aluminum hydroxide, aluminum chloride, aluminum nitride, aluminum phosphate, and aluminum sulfate. The glass is typically a silicate glass containing silicon dioxide (SiO₂) as a main component, and examples thereof include soda-lime glass, lead glass, soda-lime glass, A glass, C glass, E glass, borosilicate glass, and aluminosilicate glass. These flaky base materials may be used alone or in combination of two or more kinds thereof.

As the size of the flaky base material, the thickness thereof is, for example, in a range of 0.1 to 5 um (preferably in a range of 0.2 to 3 um), and the length thereof in the long side direction is, for example, in a range of 3 to 100 µm (preferably in a range of 5 to 50 um). The average aspect ratio (length/thickness ratio) of the flaky base material is, for example, in a range of 10 to 200 and preferably in a range of 20 to 150. In a case where the size of the flaky base material and the average aspect ratio thereof are in the above-described ranges, the coated pigment is comfortable and smooth to the touch in a coating, spreads well when applied, and exhibits good color development and gloss. Further, the average particle diameter of the flaky base material is, for example, in a range of 3 to 100 um and preferably in a range of 5 to 50 um.

Examples of a commercially available product of the flaky base material include mica particles such as SunMICA (trademark, manufactured by Sun Chemical) and IRIODIN (registered trademark, manufactured by Merck KGaA), glass particles such as GLASS FLAKE, METASHINE, and LUMIGLAN (all registered trademarks, manufactured by Nippon Sheet Glass Co., Ltd.), and aluminum particles such as HYDROLAN (registered trademark, manufactured by Eckart GmbH & Co., KG) .

The content of the flaky base material is, for example, in a range of 20% to 98% by mass and preferably in a range of 30% to 95% by mass based on the total amount of the coated pigment. Further, the content of the flaky base material is, for example, in a range of 30 to 2000 parts by mass, preferably in a range of 50 to 1500 parts by mass, and more preferably in a range of 100 to 1000 parts by mass based on 100 parts by mass of the colorant. In a case where the proportion of the flaky base material is in the above-described ranges, the coated pigment is comfortable and smooth to the touch in a coating, spreads well when applied, and exhibits good color development and gloss.

The surface of the flaky base material may be further coated with a layer containing, as a component, at least one or more selected from the group consisting of a resin, a metal, and a metal oxide. Examples of the resin include an acrylic resin, a urethane resin, a styrene resin, an ether resin, and an epoxy resin. Examples of the metal include silicon (Si), iron (Fe), aluminum (Al), sodium (Na), calcium (Ca), magnesium (Mg), potassium (K), copper (Cu), manganese (Mn), titanium (Ti), silver (Ag), gold (Au), platinum (Pt), lead (Pb), chromium (Cr), tin (Sn), molybdenum (Mo), gallium (Ga), and indium (In). Examples of the metal oxide include oxides of these metals.

A commercially available product may be used as the flaky base material, and examples thereof include "C80-1608 SunPURO Pearl Silver" (manufactured by Sun Chemical) and "Benda-Lutz MAXAL 76044EC" (manufactured by Sun Chemical).

### (Colorant)

It is preferable that the coated pigment contain at least one or more organic pigments as the colorant. Examples of the organic pigments include an azo-based pigment, a phthalocyanine-based pigment, an anthraquinone-based pigment, a perylene-based pigment, a perinone-based pigment, a quinacridone-based pigment, a thioindigo-based pigment, a dioxazine-based pigment, an isoindolinone-based pigment, a quinophthalone-based pigment, an azomethine-based pigment, a diketopyrrolopyrrole-based pigment, and an isoindoline-based pigment. Further, examples thereof include Red No. 3, Red No. 104, Red No. 106, Red No. 201, Red No. 202, Red No. 204, Red No. 205, Red No. 220, Red No. 223, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, Blue No. 404, C.I. Pigment Red 1, 2, 3, 4, 5, 6, 7, 8, 9, 12, 14, 15, 16, 17, 21, 22, 23, 31, 32, 37, 38, 41, 47, 48, 48:1, 48:2, 48:3, 48:4, 49, 49:1, 49:2, 50:1, 52:1, 52:2, 53, 53:1, 53:2, 53:3, 57, 57:1, 57:2, 58:4, 60, 63, 63:1, 63:2, 64, 64:1, 68, 69, 81, 81:1, 81:2, 81:3, 81:4, 83, 88, 90:1, 101, 101:1, 104, 108, 108:1, 109, 112, 113, 114 , 122, 123, 144, 146, 147, 149, 151, 166, 168, 169, 170, 172, 173, 174, 175, 176, 177, 178, 179, 181, 184, 185, 187, 188, 190, 193, 194, 200, 202, 206, 207, 208, 209, 210, 214, 216, 220, 221, 224, 230, 231, 232, 233, 235, 236, 237, 238, 239, 242, 243, 245, 247, 249, 250, 251, 253, 254, 255, 256, 257, 258, 259, 260, 262, 263, 264, 265, 266, 267, 268, 269, 270, 271, 272, 273, 274, 275, 276, 277, 278, 279, 280, 281, 282, 283, 284 , 285, 286, 287, 291, 295, and 296, C.I. Pigment Blue 1, 1:2, 9, 14, 15, 15:1, 15:2, 15:3, 15:4, 15:6, 16, 17, 19, 25, 27, 28, 29, 33, 35, 36, 56, 56:1, 60, 61, 61:1, 62, 63, 66, 67, 68, 71, 72, 73, 74, 75, 76, 78, and 79, C.I. Pigment Yellow 1, 2, 3, 4, 5, 10, 12, 13, 14, 15, 16, 17, 18, 24, 31, 32, 34, 35, 35:1, 36, 36:1, 37, 37:1, 40, 42, 43, 53, 55, 60, 61, 62, 63, 65, 73, 74, 77, 81, 83, 93, 94, 95, 97, 98, 100, 101, 104, 106, 108, 109, 110, 113, 114, 115, 116, 117, 118, 119, 120, 123, 126, 127, 128, 129, 139, 147, 150, 151, 152, 153, 154, 155, 156, 161, 162, 164, 166, 167, 168, 169, 170, 171, 172, 173, 174, 175, 176, 177, 179, 180, 181, 182, 185, 187, 188, 192, 193, 194, 196, 198, 199, 213, and 214, C.I. Pigment Violet 1, 1:1, 2, 2:2, 3, 3:1, 3:3, 5, 5:1, 14, 15, 16, 19, 23, 25, 27, 29, 31, 32, 37, 39, 42, 44, 47, 49, and 50, and C.I. Pigment Green 1, 2, 4, 7, 8, 10, 13, 14, 15, 17, 18, 19, 26, 36, 37, 45, 48, 50, 51, 54, 55, 58, and 59. These organic pigments may be used alone or in combination of two or more kinds thereof as appropriate depending on the desired hue.

Further, a natural dye may also be used as the colorant. Examples of the natural dye include a carotenoid-based dye, an anthocyanin-based dye, a flavonoid-based dye, a quinone-based dye, a porphyrin-based dye, a diketone-based dye, a betacyanin-based dye, and an azaphilone-based dye. Further, examples thereof include β-carotene, norbixin, bixin, capsanthin, lutein, lycopene, crocin, crocetin, astaxanthin, cyanidin acyl glucoside, cyanidin, aglycon of peonidin, anidin glucoside, delphinidin glucoside, anthocyanin, shisonin, malonylshisonin, pelargonidin acyl glucoside, cacao polyphenol, apigeninidin, luteolinidin, polymerized proanthocyanidin, safflomin, carthamin, carminic acid, laccaic acid, chlorophyll, phycocyanin, curcumin, betanin, isobetanin, ankaflavin, monascorubrin, iridoid glucoside, an ester hydrolyzate of iridoid glucoside, and eumelanin. These natural dyes may be used alone or in combination of two or more kinds thereof as appropriate depending on the desired hue.

The primary particle diameter of the colorant in the coated pigment is preferably 1.0 um or less, more preferably in a range of 0.001 to 0.8 um, and still more preferably in a range of 0.005 to 0.6 um. In a case where the primary particle diameter thereof is in the above-described ranges, the coated pigment is comfortable and smooth to the touch in a coating, spreads well when applied, and exhibits good color development and gloss.

In a case where the colorant in the coated pigment is aggregated to form secondary particles, the particle diameter thereof is preferably in a range of 0.005 um to 2.0 um and more preferably in a range of 0.01 um to 1.0 um. In a case where the secondary particle diameter thereof is in the above-described ranges, the coated pigment is comfortable and smooth to the touch in a coating, spreads well when applied, and exhibits good color development and gloss.

It is preferable that the colorant in the coated pigment be softer than the flaky base material. The degree of softness can be measured by the Mohs hardness. For example, the Mohs hardness of the flaky base material is preferably 5.0 or greater, and the Mohs hardness of the colorant is preferably 5.0 or less. When the Mohs hardness of the colorant is smaller than the Mohs hardness of the flaky base material, a smoother coated pigment can be obtained, and the coated pigment spreads well when applied and exhibits good color development and gloss.

The content of the colorant in the coated pigment is, for example, in a range of 0.5% to 50% by mass, preferably in a range of 1.0% to 40% by mass, and more preferably in a range of 1.0% to 30% by mass based on the total amount of the coated pigment. Further, the content of the colorant is, for example, in a range of 5 to 333 parts by mass, preferably in a range of 7 to 200 parts by mass, and more preferably in a range of 10 to 100 parts by mass based on 100 parts by mass of the flaky base material. In a case where the proportion of the flaky base material is in the above-described ranges, the coated pigment is comfortable and smooth to the touch in a coating, spreads well when applied, and exhibits good color development and gloss.

The coated pigment may be further coated with a layer containing, as a component, at least one or more selected from the group consisting of a resin, a metal, and a metal oxide. Examples of the resin include an acrylic resin, a urethane resin, a styrene resin, an ether resin, and an epoxy resin. Examples of the metal include silicon (Si), iron (Fe), aluminum (Al), sodium (Na), calcium (Ca), magnesium (Mg), potassium (K), copper (Cu), manganese (Mn), titanium (Ti), silver (Ag), gold (Au), platinum (Pt), lead (Pb), chromium (Cr), tin (Sn), molybdenum (Mo), gallium (Ga), and indium (In). Examples of the metal oxide include oxides of these metals.

### (Other components)

The coated pigment may contain an inorganic powder or an organic powder as other components in addition to the flaky base material and the colorant. Examples of such an inorganic powder include zirconium oxide, zinc oxide, cerium oxide, magnesium oxide, barium sulfate, calcium sulfate, magnesium sulfate, calcium carbonate, magnesium carbonate, talc, sericite, silicic acid, silicic anhydride, aluminum silicate, magnesium silicate, aluminum magnesium silicate, calcium silicate, barium silicate, strontium silicate, metal tungstates, hydroxyapatite, vermiculite, hydrargillite, bentonite, montmorillonite, hectorite, zeolite, ceramic powders, dibasic calcium phosphate, alumina, aluminum hydroxide, boron nitride, and silica.

Further, examples of the organic powder include polyamide powder, polyester powder, polyethylene powder, polypropylene powder, polystyrene powder, polyurethane powder, benzoguanamine powder, polymethyl benzoguanamine powder, tetrafluoroethylene powder, polymethyl methacrylate powder, cellulose powder, silk powder, nylon powder (nylon 12 and nylon 6), styrene acrylic acid copolymer powder, divinylbenzene styrene copolymer powder, vinyl resin powder, urea resin powder, phenol resin powder, fluorine resin powder, silicone resin powder, acrylic resin powder, melamine resin powder, epoxy resin powder, polycarbonate resin powder, microcrystalline fiber powder, rice starch, and lauroyl lysine. When the organic powder or the inorganic powder is used in the coated pigment, the content thereof is, for example, in a range of 0.1% to 10% by mass and preferably in a range of 0.5% to 5% by mass based on the total amount of the coated pigment.

The coated pigment may contain, as other components, an oily base for the purpose of stabilizing the dispersion of the colorant. Examples of such an oily base include waxes in a solid state at room temperature (25°C) and liquid oily components in a liquid state at room temperature.

Examples of the waxes include vegetable-based waxes such as candelilla wax, carnauba wax, rice wax, Japan wax, and sunflower wax, animal-based waxes such as beeswax, mineral-based waxes such as ozokerite, ceresin, and microcrystalline wax, petroleum-based waxes such as solid paraffin, and synthetic waxes such as silicone wax and synthetic beeswax.

Examples of the liquid oily components include vegetable-based oils such as olive oil, castor oil, jojoba oil, macadamia nut oil, rosa canina fruit oil, cacao butter, and lanolin, animal-based oils such as horse oil, turtle oil, boar oil, mink oil, and shark oil, hydrocarbon-based oils such as petroleum jelly, liquid paraffin, isodecane, isododecane, octyl dodecyl, and hydrogenated polyisobutene, ester oils such as isotridecyl isononanoate, isopropyl isostearate, neopentyl glycol dicaprate, isotridecyl isononanoate, glyceryl diisostearate, glyceryl triisostearate, diisostearyl malate, octyl dodecanol, and di(phytosteryl/2-octyldodecyl) N-lauroyl-L-glutamate, silicone oils such as dimethyl polysiloxane and phenyl methyl polysiloxane, dimer acid esters, dimer diol derivatives, cholesterol fatty acid esters, phytosterol fatty acid esters, polyglycerol fatty acid esters, pentaerythritol fatty acid esters, and glyceryl tri-2-ethylhexanoate. The content of the wax or the liquid oily component in the coated pigment is, for example, in a range of 0.1% to 10% by mass and preferably in a range of 0.5% to 5% by mass based on the total amount of the coated pigment.

### [Method of producing coated pigment]

In a method of producing the coated pigment, for example, a wet method, a vapor phase method, or a solid phase method can be used as a method of coating the surface of the flaky base material with the colorant. Examples of the wet method include a method of using chemical adsorption in a solvent. Examples of the vapor phase method include a method of directly coating the surface of the base material using sublimation. Examples of the solid phase method include a method of using a dry particle compounding device. In the production method, an adsorbent may be used.

According to the method of producing the coated pigment using the wet method, for example, the coated pigment can be obtained through acid-base chemical adsorption by mixing a flaky base material having a surface subjected to a carboxylic acid treatment, a colorant, and an amine compound in an organic solvent and filtering the mixture. The organic solvent used here may be a solvent that does not dissolve the flaky base material, and the solvent may or may not dissolve the colorant.

The colorant is typically uniformly applied by bringing a solution or dispersion of the colorant in the organic solvent into contact with the flaky base material.

According to the method of producing the coated pigment using the vapor phase method, for example, the coated pigment can be obtained by placing the flaky base material and the colorant in an evaporator, raising the temperature and reducing the pressure while rotating the evaporator, maintaining the rotation for a certain period of time, and cooling the mixture while gradually returning the pressure to normal pressure. The colorant is sublimated by raising the temperature and reducing the pressure, and the colorant in a gaseous state is uniformly applied to the flaky base material.

According to the method of producing the coated pigment using the solid phase method, for example, the coated pigment can be obtained by premixing a colorant such as an organic pigment with the flaky base material so that the colorant and the flaky base material are uniformly present, and then stirring the colorant and the flaky base material using a dry particle compounding device or the like to apply compression, shear, and impact so that the colorant is extended and fixed to the surface of the flaky base material.

As the dry particle compounding device, the product name "NOBILTA (registered trademark) NOB" (manufactured by Hosokawa Micron Corporation) can be preferably used. The peripheral speed of stirring in the dry particle compounding device is, for example, in a range of 5 to 20 m/sec and preferably in a range of 10 to 20 m/sec (for example, in a range of 1000 to 5000 rpm and preferably in a range of 2000 to 4000 rpm). The time for the stirring treatment is, for example, in a range of 1 to 10 minutes and preferably in a range of 2 to 5 minutes.

As a result of research conducted by the present inventors, it has been found that, although the peripheral speed of stirring in the related art (for example, PTL 1) is set to 30 m/sec or greater, the force applied to the premixed composite material is extremely large when the peripheral speed of stirring is set to 30 m/sec or greater, which may result in an unsmooth surface condition due to cracking of the flaky base material from the edge thereof. In the present invention, a coated pigment with unprecedented surface smoothness can be obtained under appropriate conditions.

### [Cosmetic material, ink, coating material, toner, or molded article]

The cosmetic material, the ink, the coating material, the toner, or the molded article in the present invention is not particularly limited as long as the coated pigment is contained. In addition to the coated pigment, common components suitable for the individual applications and purposes can be blended as appropriate.

Examples of the cosmetic material include a foundation (such as a face color or a concealer), a cosmetic base (such as a makeup base or a pre-makeup base), a makeup powder (face powder), a lipstick (such as a lipstick, a lip rouge, a lip color, a lip pencil, a muddy colored lipstick or rouge, a lip gloss, or a lip liner), an eye makeup (such as an eye shadow, an eye color, an eyeliner, a blackened eyebrow, an eyebrow pencil, an eyebrow brush, mascara, or an eyelash cosmetic material), a cheek cosmetic material (such as a blusher, a cheek color, or a cheek rouge), a nail cosmetic material (such as a nail enamel, a manicure, a nail color, a nail polish, a pedicure, a nail lacquer, a top coat, or a base coat), and a hair coloring material (such as a hair dye, a hair color spray, a hair color stick, a color rinse, or a hair manicure).

As the ink, particularly a printing ink can be used, and examples thereof include a lithographic (offset) ink, a letterpress ink, an intaglio (gravure) ink, a stencil (screen) ink, a flexographic ink, a UV curing ink, an aqueous ink, an oil-based ink, a vegetable oil ink, a newspaper ink, and an ink jet ink.

Examples of the coating material include synthetic resin coating materials such as a powder coating material, an aqueous (water-based) coating material, an epoxy resin coating material, a urethane resin coating material, a fluorocarbon resin coating material, a polyester resin coating material, a melamine resin coating material, and an acrylic resin coating material.

The toner is a micro-sized powder, for use in a laser printer or a copying machine, obtained by depositing color particles on plastic particles having electrostatic properties, and the coated pigment can be used as the color particles.

The molded article is molded by using a resin containing the coated pigment as a plastic colorant. The plastic colorant may be in any form, such as a masterbatch (MB), a colored pellet, a colored compound, a dry color, a paste color, or a liquid masterbatch.

### EXAMPLES

Hereinafter, the present invention will be described in more detail with reference to examples and comparative examples. In the description of the present examples, "parts" denotes "parts by mass". Coating-material-applied paper (sample) was prepared by the following method using coated pigments of Examples 1 to 8 and Comparative Examples 1 to 6, and evaluations were performed by the following methods. The evaluation results are listed in Table 1.

### [Example 1]

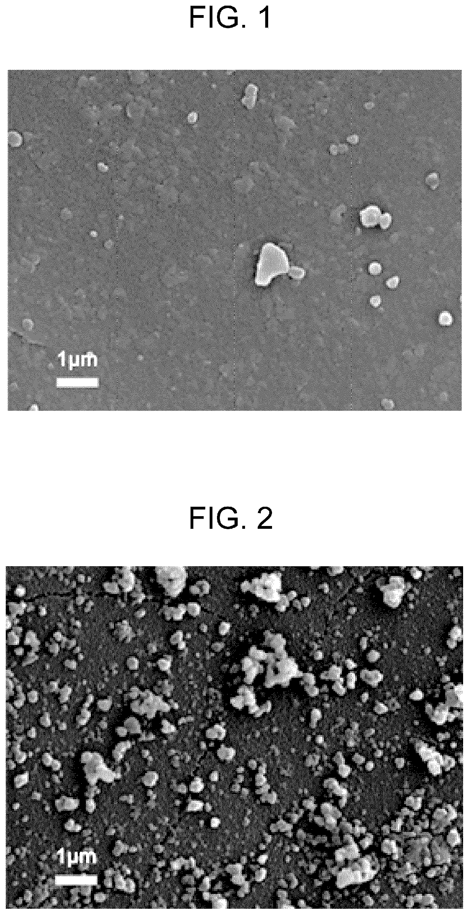

4.0 parts of a commercially available Red No. 104 (C146623, manufactured by Sun Chemical) and 36.0 parts of mica particles A coated with titanium oxide (pinkish mica particles) were premixed. Next, the mixture was treated using a dry particle compounding device (the product name "NOBILTA (registered trademark) NOB", manufactured by Hosokawa Micron Corporation; a blade having a diameter of 86 mm was used) at 3000 rpm for 3 minutes and was then taken out to obtain a coated pigment (1). A SEM image of the coated pigment (1) is shown in Fig. 1.

0.87 parts of the coated pigment (1) obtained above, 12.6 parts of ACRYDIC A-801-P (manufactured by DIC Corporation), 3.9 parts of DESMODUR N-75 (manufactured by Sumika Covestro Urethane Co., Ltd.), 1.6 parts of xylene, and 1.0 parts of ethyl acetate were blended and dispersed with a planetary mill for 3 minutes to prepare a coating material composition (1). The resulting coating material composition (1) was applied to black paper with a 10 mil applicator to obtain coating-material-applied paper (1).

### [Example 2]

A coated pigment (2) and coating-material-applied paper (2) were obtained in the same manner as in Example 1 except that Red No. 104 was replaced with Red No. 202 (C19-7720, manufactured by Sun Chemical) and the mica particles A were replaced with mica particles B coated with titanium oxide (reddish mica particles) in Example 1.

### [Example 3]

A coated pigment (3) and coating-material-applied paper (3) were obtained in the same manner as in Example 1 except that 4.0 parts of Red No. 104 was replaced with 5.5 parts of Blue No. 1 (C39-4433, manufactured by Sun Chemical) and the mica particles A were replaced with 34.5 parts of mica particles C coated with titanium oxide (bluish mica particles) in Example 1.

### [Example 4]

A coated pigment (4) and coating-material-applied paper (4) were obtained in the same manner as in Example 1 except that Red No. 104 was replaced with a quinacridone pigment (FASTOGEN SUPER MAGENTA RTS, manufactured by DIC Corporation) in Example 1.

### [Example 5]

A coated pigment (5) and coating-material-applied paper (5) were obtained in the same manner as in Example 1 except that Red No. 104 was replaced with a copper phthalocyanine pigment (FASTOGEN BLUE TGR-SD, manufactured by DIC Corporation, average particle diameter: 0.05 µm) and the mica particles A were replaced with the mica particles C coated with titanium oxide in Example 1.

### [Example 6]

A coated pigment (6) and coating-material-applied paper (6) were obtained in the same manner as in Example 1 except that Red No. 104 was replaced with a copper phthalocyanine pigment (BLUE CRUDE B-8/LC, manufactured by DIC Corporation, average particle diameter: 3 µm) and the mica particles A were replaced with the mica particles C coated with titanium oxide in Example 1.

### [Example 7]

A coated pigment (11) and coating-material-applied paper (11) were obtained in the same manner as in Example 1 except that the mica particles A were replaced with aluminum particles coated with titanium oxide (MAXAL 76044EC, manufactured by Sun Chemical) and the mixture was treated using a dry particle compounding device at 3000 rpm for 10 minutes in Example 1.

### [Example 8]

A coated pigment (12) and coating-material-applied paper (12) were obtained in the same manner as in Example 1 except that Red No. 104 was replaced with Blue No. 1 (C39-4433, manufactured by Sun Chemical), the mica particles A were replaced with borosilicate glass particles coated with titanium oxide (Reflecks Dimensions Shimmering White G130Z, Colors & Effects Japan Ltd.), and the mixture was treated using a dry particle compounding device at 2000 rpm for 5 minutes in Example 1.

### [Comparative Example 1]

A coated pigment (7) and coating-material-applied paper (7) were obtained in the same manner as in Example 1 except that NOBILTA was replaced with AMS-MINI in Example 1. A SEM image of the coated pigment (7) is shown in Fig. 2.

### [Comparative Example 2]

A coated pigment (8) and coating-material-applied paper (8) were obtained in the same manner as in Example 2 except that NOB-MINI was replaced with AMS-MINI and the mixture was treated at 1500 rpm for 1 minute in Example 2.

### [Comparative Example 3]

A coated pigment (9) and coating-material-applied paper (9) were obtained in the same manner as in Example 3 except that NOB-MINI was replaced with AMS-MINI in Example 3.

### [Comparative Example 4]

A coated pigment (10) and coating-material-applied paper (10) were obtained in the same manner as in Example 4 except that NOB-MINI was replaced with AMS-MINI in Example 4.

### [Comparative Example 5]

A coated pigment (13) and coating-material-applied paper (13) were obtained in the same manner as in Example 7 except that the dry particle compounding time was changed from 10 minutes to 3 minutes in Example 7.

### [Comparative Example 6]

A coated pigment (14) and coating-material-applied paper (14) were obtained in the same manner as in Example 8 except that the rotation speed in the dry particle compounding treatment was changed from 2000 rpm to 3000 rpm and the treatment time was changed from 5 minutes to 1 minute in Example 8.

### [Evaluation: method of calculating coarse particle area fraction]

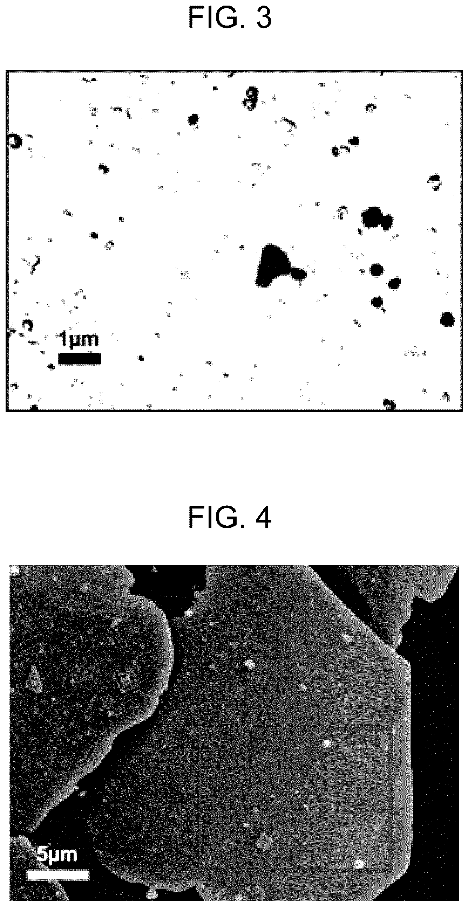

An image was captured at a magnification of 10000 times with a scanning electron microscope (VE-7800, KEYENCE Corporation) such that only the surface of the coated pigment was shown in the image. The image was subjected to binarization processing by adjusting the threshold value such that coarse particles were visually recognizable using image processing software (ImageJ), and the occupied area fraction of the coarse particles in a region excluding 5% of the top, bottom, left, and right of the image was calculated. The same processing was performed on ten images for each kind of coated pigment, and the average value was determined as the coarse particle area fraction. For example, in a case where the coated pigment (1) of Example 1 is subjected to binarization processing into black and white, the results are as shown in Fig. 3.

### [Evaluation: measurement of MIU]

The friction coefficient, i.e., the MIU value, was measured using a friction feel tester (KES-SE; Kato Tech Co., Ltd.) by placing a sensor on a sample spread on a sample table and sliding the sensor. Further, data for a length of 20 mm was employed by cutting data for a length of initial 5 mm and a length of final 5 mm. A surface with a smaller MIU value is smoother to the touch.

### [Evaluation: measurement of sparkle intensity]

The resulting coating-material-applied paper was subjected to three-point color measurement using a multi-angle colorimeter (BYK-maci), and the average Si value (sparkle intensity) at 45° was obtained.

**[Table 1]**

| | | Coarse particle area fraction | Average MIU value | Average Si value |
|---|---|---|---|---|
| Example 1 | Coated pigment (1) | 2.07% | 0.389 | 8.6 |
| Comparative Example 1 | Coated pigment (7) | 19.0% | 0.471 | 8.0 |
| Example 2 | Coated pigment (2) | 2.68% | 0.687 | 9.2 |
| Comparative Example 2 | Coated pigment (8) | 46.3% | 0.770 | 2.4 |
| Example 3 | Coated pigment (3) | 2.01% | 0.382 | 10.2 |
| Comparative Example 3 | Coated pigment (9) | 19.8% | 0.444 | 10.2 |
| Example 4 | Coated pigment (4) | 0.58% | 0.567 | 11.3 |
| Comparative Example 4 | Coated pigment (10) | 11.6% | 0.595 | 7.4 |
| Example 5 | Coated pigment (5) | 1.57% | 0.490 | 12.9 |
| Example 6 | Coated pigment (6) | 4.07% | 0.577 | 12.0 |
| Example 7 | Coated pigment (11) | 5.23% | 0.574 | 13.6 |
| Comparative Example 5 | Coated pigment (13) | 14.16% | 0.594 | 13.1 |
| Example 8 | Coated pigment (12) | 8.69% | 0.662 | 117.7 |
| Comparative Example 6 | Coated pigment (14) | 20.17% | 0.716 | 99.5 |

Based on comparisons between Example 1 and Comparative Example 1, between Example 2 and Comparative Example 2, between Example 3 and Comparative Example 3, between Example 4 and Comparative Example 4, between Example 7 and Comparative Example 5, and between Example 8 and Comparative Example 6, in which the same colorants were used, it was found that the average MIU values in the examples, in which the coarse particle area fractions were smaller, were smaller, and the surface was more comfortable to the touch. Further, it was found that the Si values in the examples were greater than or equal to the Si values in the comparative examples, and thus the glittering appearance indicated by the sparkle intensity was maintained or improved. Further, when the surfaces of the pigments of Example 1 (coated pigment (1); Fig. 1) and Comparative Example 1 (coated pigment (7); Fig. 2) were observed in magnified SEM images, it was found that the surface in Fig. 1 was clearly smoother and had fewer coarse particles than the surface in Fig. 2.

In Example 5 and Example 6, the particle diameters of the primary particles were different from each other, but pigments with sufficiently small average MIU values and large Si values were obtained.

## Claims

1. A pigment which is a coated pigment formed by coating a surface of a flaky base material made of at least one metal or metal oxide with a colorant, wherein the surface of the flaky base material has a coarse particle area fraction of 9% or less.

2. The pigment according to claim 1, wherein the flaky base material is at least one or more selected from the group consisting of mica, aluminum, alumina, and glass.

3. The pigment according to claim 1 or 2, comprising at least one or more organic pigments as the colorant.

4. The pigment according to any one of claims 1 to 3, wherein the surface of the flaky base material is further coated with a layer containing, as a component, at least one or more selected from the group consisting of a resin, a metal, and a metal oxide.

5. The pigment according to any one of claims 1 to 4, wherein the pigment is further coated with a layer containing, as a component, at least one or more selected from the group consisting of a resin, a metal, and a metal oxide.

6. A cosmetic material, an ink, a coating material, a toner, or a molded article, comprising the pigment according to any one of claims 1 to 5.
